# EUROPEAN PATENT SPECIFICATION

(11) **EP 0 703 210 B1**
(45) Date of publication and mention of the grant of the patent: **31.03.1999**
(21) Application number: 95114795.8
(22) Date of filing: 20.09.1995
(51) Int. Cl.: C07C 29/20, C07C 35/08

(54) **Process for producing 2,6-di-tert-alkylcyclohexanol**
Verfahren zur Herstellung von 2,6-Di-tert-alkylcyclohexanol
Procédé pour la préparation de 2,6-di-tert-alkylcyclohexanol

(30) Priority: 20.09.1994 JP 224864/94
(43) Date of publication of application: 27.03.1996
(73) Proprietor: FUJI PHOTO FILM CO., LTD., Kanagawa-ken (JP)
(72) Inventor: Matsuoka, Koshin, c/o Fuji Photo Film Co., Ltd., Kanagawa (JP)
(74) Representative: Hansen, Bernd, Dr. Dipl.-Chem.

(56) References cited:
- GB-A- 2 016 449
- US-A- 2 840 599
- US-A- 4 551 564
- US-A- 4 751 214
- CHEMICAL ABSTRACTS, vol. 74, no. 25, 21 June 1971 Columbus, Ohio, US; abstract no. 141098c, page 533; & JP-A-45 035 300 (MITSUI TOATSU CHEMICALS) 11 November 1970
- COMPTES RENDUS DES SEANCES DE L'ACADEMIE DES SCIENCES, SERIE C: SCIENCES CHIMIQUES, vol. 291, no. 8, 1980 MONTREUIL, FR, pages 219-221, R. LAMARTINE, ET AL.: 'Hydrogénation du méthyl-3 isopropyl-6 phénol solide. Influence du catalyseur et du support sur la sélectivité de la réaction; comparaison avec les résultats obtenus en solution'
- JOURNAL OF THE AMERICAN CHEMICAL SOCIETY, vol. 79, no. 18, 24 September 1957 WASHINGTON, DC, US, pages 5019-5023, T.H. COFFIELD, ET AL.: 'Some reactions of 2,6-dialkylphenols'

## Description

This invention relates to a process for producing a 2,6-di-tert-alkylcyclohexanol which is applicable to an element for photographing such as a synthetic intermediate of coupler for color photographing and a dispersing medium for photographing, a solvent and a dye intermediate, as well as a synthetic intermediate for medicines and agricultural chemicals.

### BACKGROUND OF THE INVENTION

In recent years, a coupler for use in a silver halide color photographic material has been required to possess very high performances. For example, it is required to have good color tone, a high coupling activity, a high stability to components of a fixer for formed dyes or a blixer, a little variation of color forming density even if the compositions of these processing solutions are varied, and a good balance of excellent color image fastness after processing (fastness at a low density region and fastness at a high density region), etc.

The inventors have studied intensively in order to attain such a object, and have found that a pyroloazole coupler in which a cycloalkoxycarbonyl group possessing bulky substituents in both ortho positions is incorporated excels in the above-mentioned performances. The pyroloazole coupler in which a cycloalkoxycarbonyl group possessing bulky substituents in both ortho positions is incorporated is produced from a cyclic alcohol possessing bulky substituents in both ortho positions as a raw material.

An example of the cyclic alcohol possessing bulky substituents in both ortho includes a 2,6-di-tert-alkylcyclohexanol. As an example of synthesizing such a 2,6-di-tert-alkylcyclohexanol, a process which comprises catalytically hydrogenating 2,6-di-tert-butylphenol in the presence of a nickel catalyst to obtain 2,6-di-tert-butylhexanone, and further reducing it with aluminum lithium hydride to obtain 2,6-di-tert-butylcyclohexanol is described in Journal of American Chemical Society, Vol. 79, pp 5019-5023 (1957).

However, this process requires two steps for synthesizing a corresponding cyclohexanol from a raw material phenol as a raw material. In addition, the reaction with aluminum lithium hydride is not suitable for mass supply of product in terms of cost and safety.

According to Journal of American Chemical Society, Vol. 69, pp. 2414-2415 (1947) and the above-described Journal of American Chemical Society, Vol. 79, pp 5019-5023 (1957), an alkyl-substituted phenol can be generally derived to a corresponding cyclohexanol by catalytic hydrogenation with a catalyst such as nickel, but in the case of a phenol in which both of the 2- and 6-positions are substituted by tert-alkyl groups as shown in formula (I), this process dose not give a cyclohexanol but gives a cyclohexanone.

As described above, the development of a synthesizing process which can produce a 2,6-di-tert-alkylcyclohexanol at a low cost on a large scale has been strongly desired.

### SUMMARY OF THE INVENTION

An object of the present invention is to provide a process which can produce a large amount of a 2,6-di-tert-alkylcyclohexanol from a 2,6-di-tert-phenol in a high yield at one stage.

From such a viewpoint, the present inventors have made a various studies of the catalytic hydrogenation of 2,6-di-tert-alkylcyclohexanol and, as a result, have accomplished the present invention.

To be specific, the object of the present invention is attained by the process as described below:
(1) A process for producing a 2,6-di-tert-alkylcyclohexanol represented by formula (II), which comprises catalytically hydrogenating a 2,6-di-tert-alkylphenol represented by formula (I) in the presence of a rhodium or ruthenium catalyst: 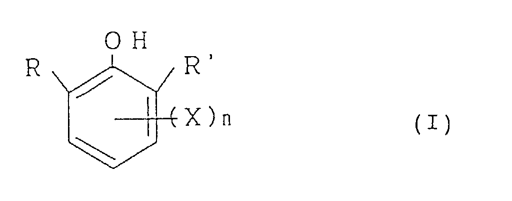 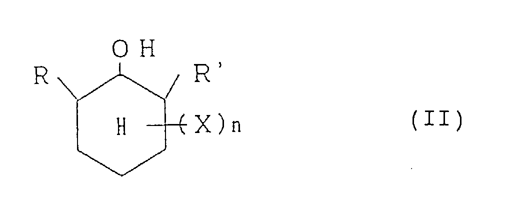 wherein R and R' are independently represented by formula (III) 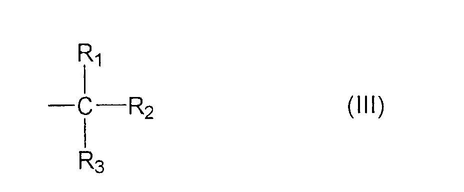 wherein R₁, R₂ and R₃ may be the same or different and represent a substituent, which may be connected to each other to form a ring, X represents a substituent, and n is an integer from 0 to 3.
(2) A process according to the above Item (1), wherein the catalytic hydrogenation reaction is carried out in the absence of any solvent.
(3) A process according to the above Item (1) or (2), wherein the catalytic hydrogenation reaction is carried out at a temperature of from 80°C to 240°C.
(4) A process according to the above Item (1), (2), or (3), wherein the catalytic hydrogenation reaction is carried out in a reactor at a hydrogen pressure of from 15 kg/cm² to 200 kg/cm².

### DETAILED DESCRIPTION OF THE INVENTION

The present invention will now be described below in detail. First, formulae (I) and (II) will be explained.

In formulae (I) and (II), X represents a substituent except for a hydrogen atom. Examples of X include aliphatic groups (e.g. those having 1 to 36 carbon atoms such as straight or branched chain alkyl groups, aralkyl groups, alkenyl groups, cycloalkyl groups, and cycloalkenyl groups having 1 to 36 carbon atoms; more specifically, such as methyl, ethyl, propyl, isopropyl, t-butyl, tridecyl, cyclohexyl, 4-pentadecyloxybenzyl, hexadecyloxycarbonylethyl, 2-ethoxytridecyl, trifluoromethyl, cyclopentyl, and 3-(2,4-di-t-amylphenoxy)propyl), aryl groups (preferably those having 6 to 36 carbon atoms such as phenyl, naphthyl, 4-t-butylphenyl, 2,4-di-t-amylphenyl, 4-tetradecanamidephenyl, and 3-(2,4-di-t-amylphenoxyacetamide)phenyl), heterocyclic groups (preferably those having 1 to 30 carbon atoms such as 3-pyridyl, 2-furyl, 2-pyridyl, and 2-pyrimidinyl), alkoxy groups (preferably those having 1 to 30 carbon atoms such as methoxy, ethoxy, 2-methylmethoxy, and 2-dodecyloxyethoxy), aryloxy groups (preferably those having 6 to 30 carbon atoms such as phenoxy, 2-methylphenoxy, 4-tert-butylphenoxy, 2,4-di-tert-amylphenoxy, 2-chlorophenoxy, 4-cyanophenxy, 3-nitrophenoxy, 3-t-butyloxycarbamoylphenoxy, and 3-methoxycarbamoylphenoxy), heterocyclic oxy groups (preferably those having 1 to 30 carbon atoms such as 2-benzimidazolyloxy, and l-phenyltetrazole-5-oxy, 2-tetrahydropyranyloxy), alkyl-, aryl- or heterocyclic acyloxy groups (preferably those having 2 to 30 carbon atoms such as acetoxy and hexadecanoyloxy), carbamoyloxy groups (preferably those having 1 to 30 carbon atoms such as N-ethylcarbamoyloxy and N-phenylcarbamoyloxy), silyloxy groups (preferably those having 1 to 30 carbon atoms such as trimethylsilyloxy and dibutylmethylsilyloxy), acylamino groups (preferably those having 2 to 30 carbon atoms such as acetamide, benzamide, tetradecanamide, 2-(2,4-di-t-amylphenoxy)acetamide, isopentadecanamide, 2-(2,4-di-t-amylphenoxy)butanamide, and 4-(3-t-butyl-4-hydroxyphenoxy)butanamide), alkylamino groups (preferably those having 1 to 30 carbon atoms such as methylamino, butylamino, dodecylamino, dimethylamino, diethylamino, and methylbutylamino), arylamino groups (preferably those having 6 to 30 carbon atoms such as phenylamino, 2-chloroanilino, 2-chloro-5-tetradecanamideanilino, N-acetylanilino, 2-chloro-5-[(α-2-tert-butyl-4-hydroxyphenoxy)dodecylamide]anilino, and 2-chloro-5-dodecyloxycarbonylanilino), ureide groups (preferably those having 2 to 30 carbon atoms such as methylureide, phenylureide, N,N-dibutylureide, and dimethylureide), alkenyloxy groups (preferably those having 2 to 30 carbon atoms such as 2-propenyloxy), formyl group, alkyl-, aryl- or heterocyclic acyl groups (preferably those having 1 to 30 carbon atoms such as acetyl, benzoyl, 2,4-di-tert-amylphenylacetyl, 3-phenylpropanoyl, and 4-dodecyloxybenzoyl), alkyl-, aryl- or heterocyclic oxycarbonyl groups (preferably those having 2 to 30 carbon atoms such as methoxycarbonyl, butoxycarbonyl, dodecyloxycarbonyl, octadecyloxycarbonyl, phenyloxycarbonyl, and 2-pentadecyloxycarbonyl), alkyl-, aryl- or heterocyclic oxycarbonylamino groups (preferably those having 2 to 30 carbon atoms such as methoxycarbonylamino, tetradecyloxycarbonylamino, phenoxycarbonylamino, and 2,4-di-tert-butylphenoxycarbonylamino), carbamoyl groups (preferably those having 1 to 30 carbon atoms such as N-ethylcarbamoyl, N,N-dibutylcarbamoyl, N-(2-dodecyloxyethyl)carbamoyl, N-methyl-N-dodecylcarbamoyl, and N-[3-(2,4-di-tert-amylphenoxy)propyl]carbamoyl), phosphonyl groups (preferably those having 1 to 30 carbon atoms such as phenoxyphosphonyl, octyloxyphosphonyl, and phenylphosphonyl), imide groups (preferably those having 1 to 30 carbon atoms such as N-succinimide, hydantoinyl, N-phthalimide, and 3-octadecenylsuccinimide), azolyl groups (such as imidazolyl, pyrazolyl, 3-chloro-pyrazol-1-yl, and triazolyl), halogen atoms (such as chlorine atom and bromide atom), hydroxy group, cyano group, carboxy group, nitro group, unsubstituted amino group, and the like.

X is preferably a straight or branched chain alkyl group having 1 to 15 carbon atoms, more preferably having 1 to 8 carbon atoms; an aralkyl group having 7 to 15 carbon atoms, more preferably having 7 or 8 carbon atoms; a cycloalkyl group having 3 to 15 carbon atoms, more preferably having 5 to 8 carbon atoms; or an aryl group having 6 to 15 carbon atoms, more preferably having 6 to 8 carbon atoms. In particular, X is preferably methyl group, ethyl group, n- propyl group, i-propyl group, n-butyl group, s-butyl group, t-amyl group, t-octyl group, phenyl group, or cyclohexyl group, and most preferably X is methyl group. n is preferably 0 or 1, and when n is 1, X preferably exists on the para-position to the hydroxyl group of formulae (I) and (II).

In formulae (I) and (II), R and R' are independently represented by formula (III). 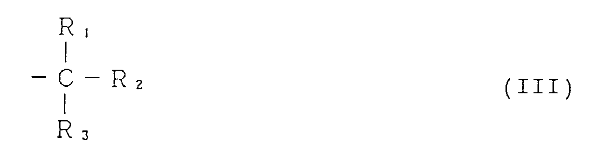 wherein R₁, R₂, and R₃ are independently a substituent except for a hydrogen atom. The substituents which can be mentioned herein are the substituents listed in X provided that it can be connected by a carbon atom.

R₁, R₂, and R₃ each is preferably a straight or branched chain alkyl group having 1 to 15 carbon atoms, more preferably having 1 to 8 carbon atoms; an aralkyl group having 7 to 15 carbon atoms, more preferably having 7 or 8 carbon atoms; a cycloalkyl group having 3 to 15 carbon atoms, more preferably having 5 to 8 carbon atoms; or an aryl group having 6 to 15 carbon atoms, more preferably having 6 to 8 carbon atoms. In particular, a straight or branched chain alkyl group having 1 to 4 carbon atoms is preferred as R₁, R₂, and R₃, with methyl group being most preferable. R₁, R₂, and R₃ may be the same or different from each other and may be connected to each other to form a ring.

Preferred examples of R and R' include tert-butyl group, tert-amyl group, tert-octyl group, 1-methylcyclohexyl group, and tert-butyl group is most preferred as R and R'.

R and R' existing on both of the ortho positions relative to the hydroxy group of formulae (I) and (II) may be the same or different from each other, but it is more preferable that the two substituents are the same.

When a substituent which is active to catalytic hydrogenation such as a multiple bond or an aromatic ring exists on R and R' in formula (I), as a result of the substituent undergoing the catalytic hydrogenation, this substituent in (II) may become a substituent different from that in formula (I).

In formula (I) and (II), n is an integer of from 0 to 3. When n is 2 or 3, X may be the same or different and a plurality of X may be connected to each other to form a ring. Similar to R and R' described previously, when a substituent which is active to catalytic hydrogenation such as a multiple bond or an aromatic ring exists on X in formula (I), as a result of the substituent undergoing the catalytic hydrogenation, the substituent in (II) may become a substituent different from that in formula (I). In addition, when X is substituted into a hydrogen atom by catalytic hydrogenation, n may become a different integer. n is preferably 1.

Preferable compounds represented by formula (I) or (II) according to the present invention are those in which R₁, R₂ and R₃ in formula (III) are independently an alkyl group having 1 to 15 carbon atoms, an aralkyl group having 7 to 15 carbon atoms, a cycloalkyl group having 3 to 15 carbon atoms, or an aryl group having 6 to 15 carbon atoms; X is an alkyl group having 1 to 15 carbon atoms, an aralkyl group having 7 to 15 carbon atoms, a cycloalkyl group having 3 to 15 carbon atoms, or an aryl group having 6 to 15 carbon atoms; n is 1; and X exists on the para position to the hydroxyl group of formulae (I) and (II). More preferable compounds of formula (I) or (II) are those in which both of R and R' are tert-butyl group, tert-amyl group, tert-octyl group or 1-methylcyclohexyl group; X is methyl group, ethyl group, n-propyl group, iso-propyl group, n-butyl group, sec-butyl group, tert-amyl group, tert-octyl group, phenyl group or cyclohexyl group; n is 1; and X exists on the para position to the hydroxyl group of formulae (I) and (II). In particular, compounds in which X is methyl group are preferable.

Typical examples of compounds represented by formula (I) or (II) according to the present invention will now be described, but the present invention is not restricted thereto.

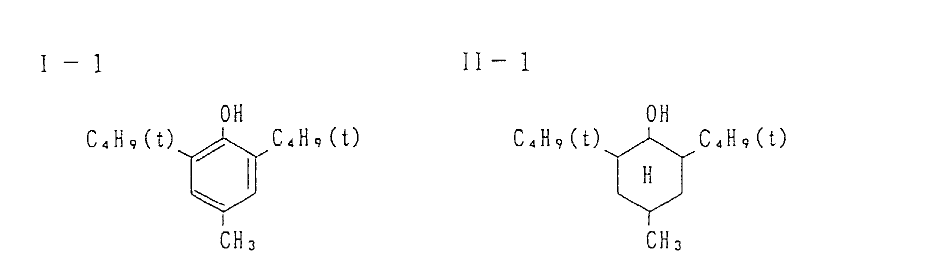

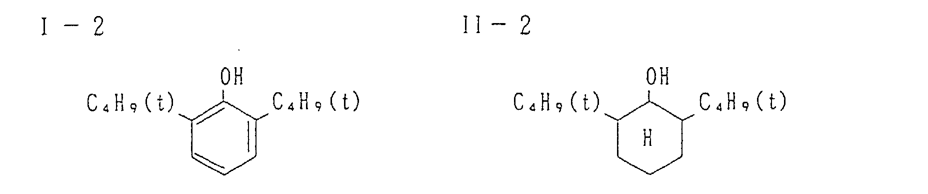

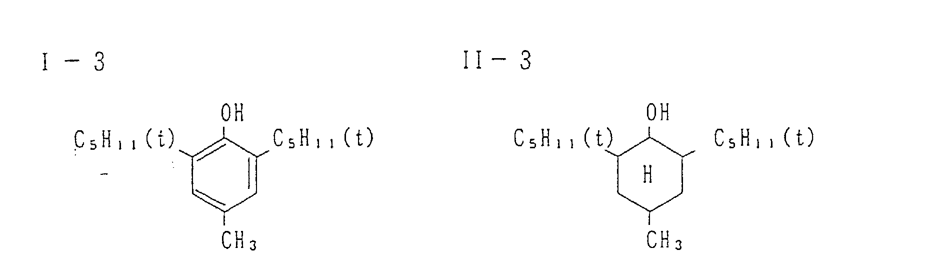

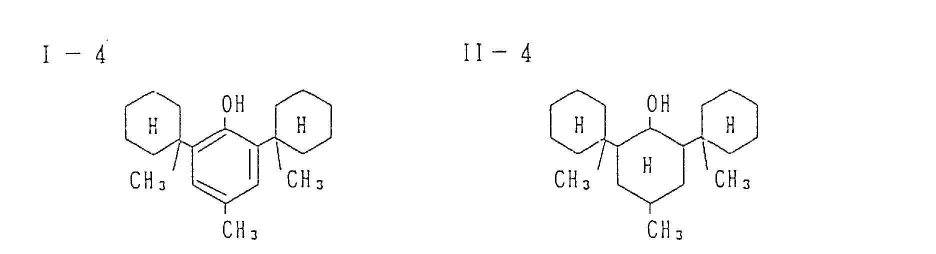

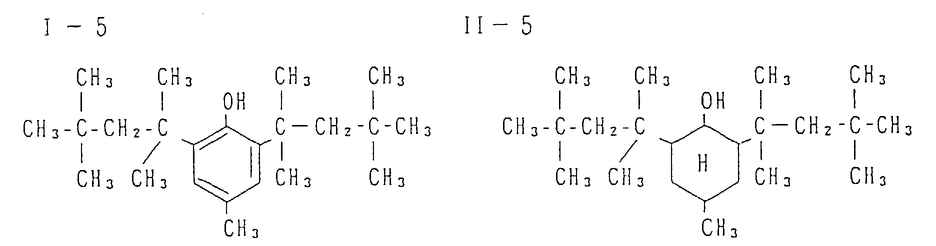

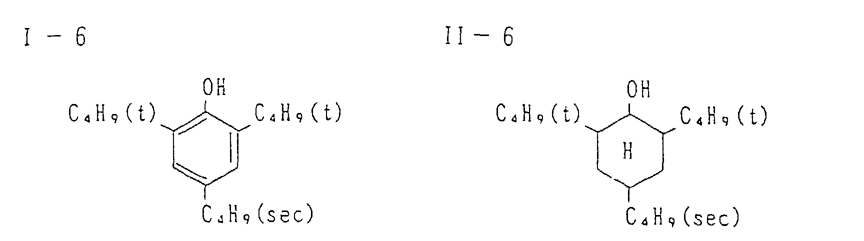

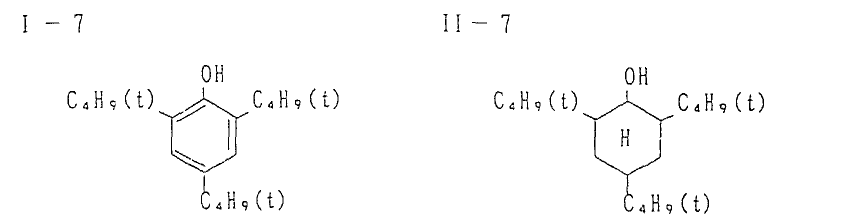

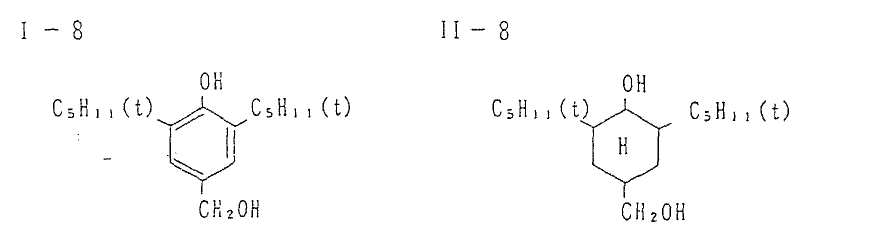

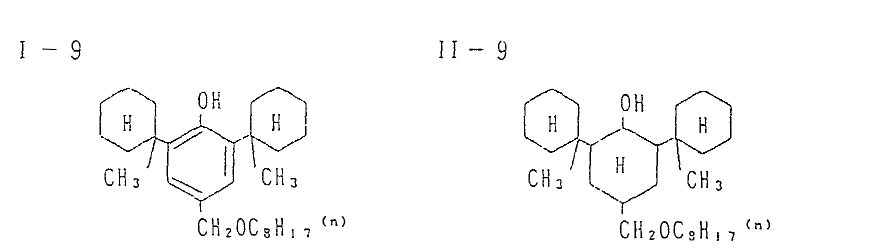

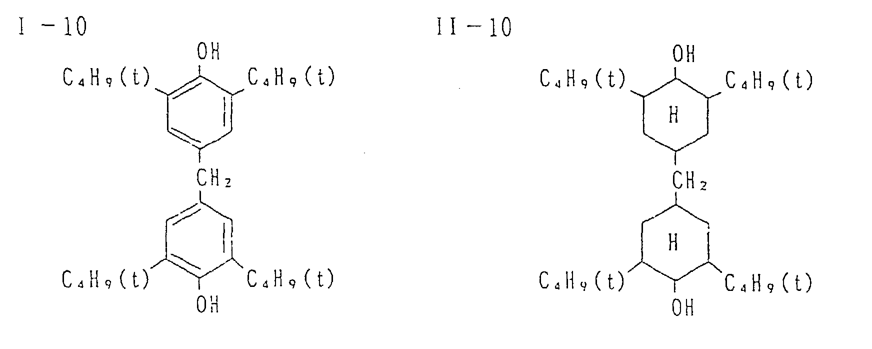

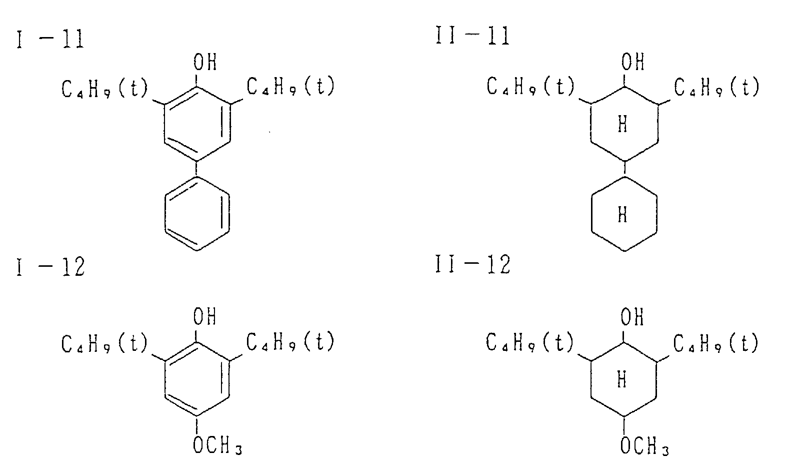

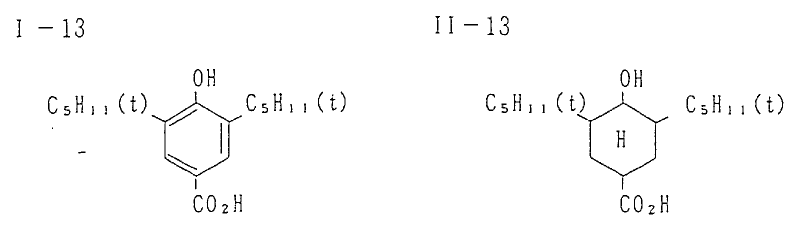

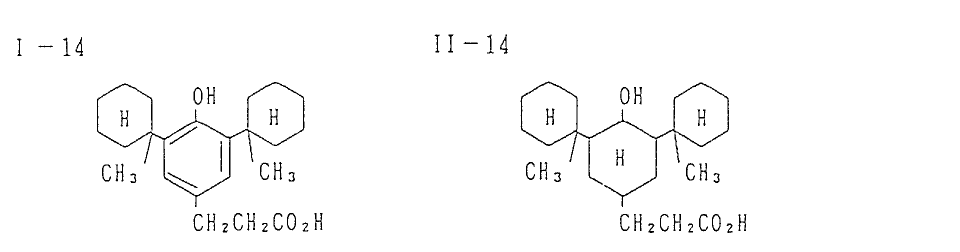

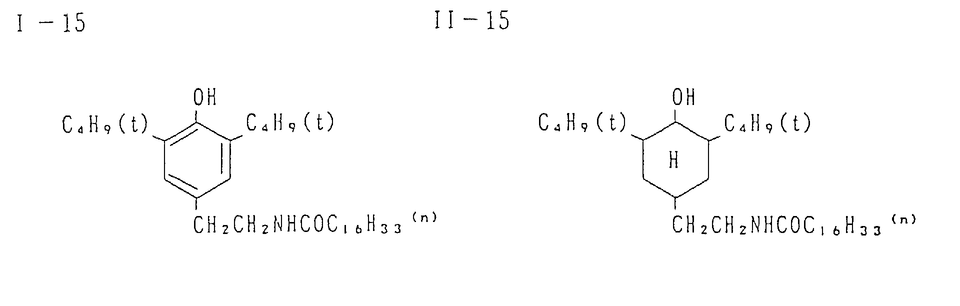

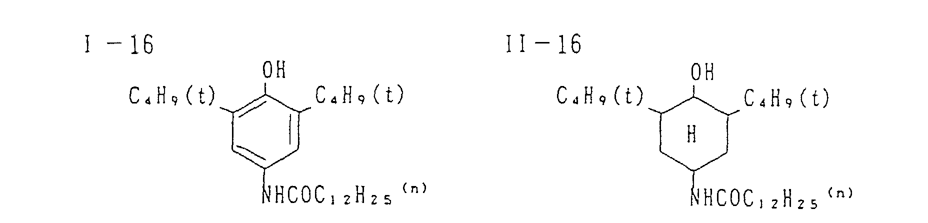

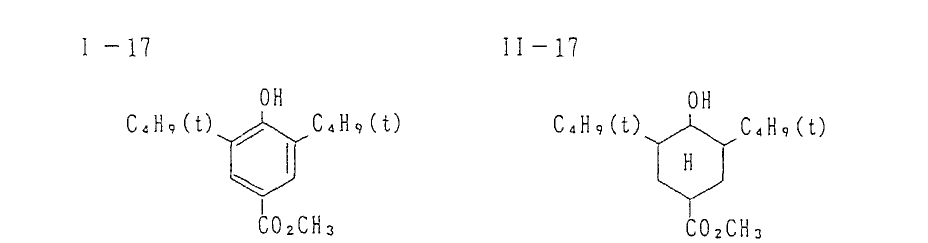

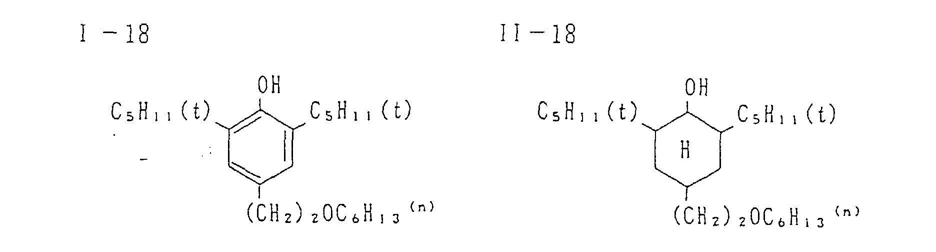

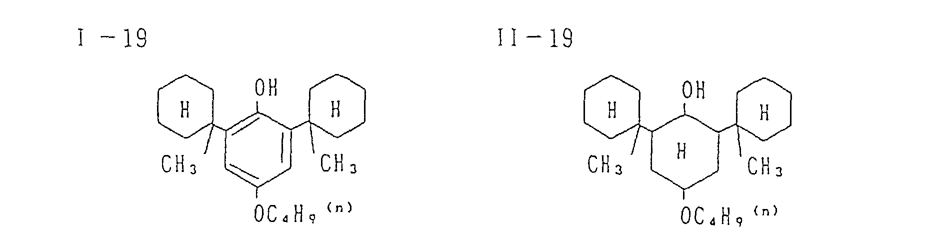

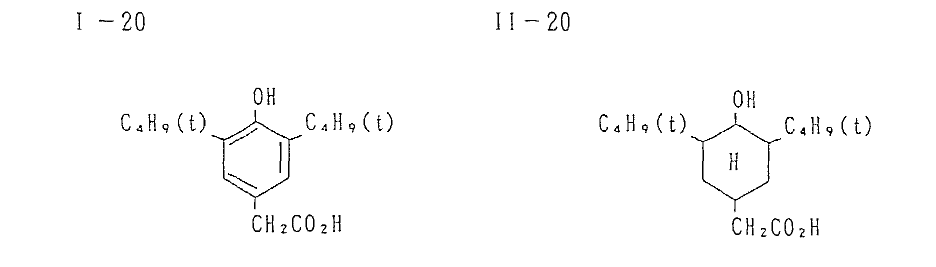

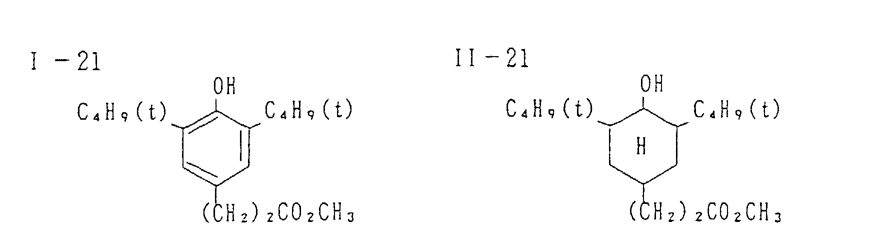

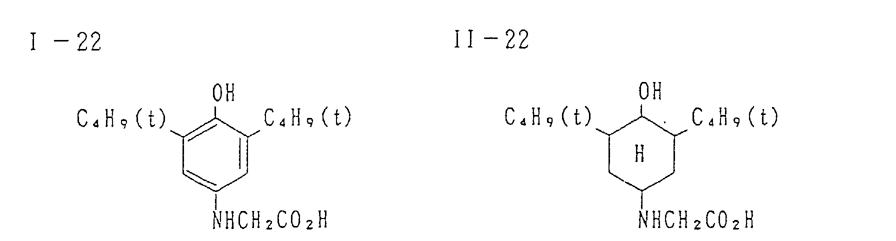

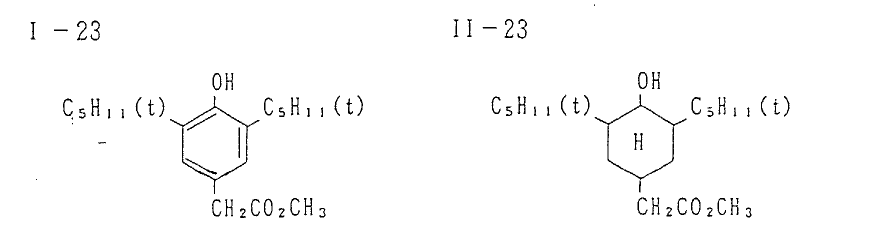

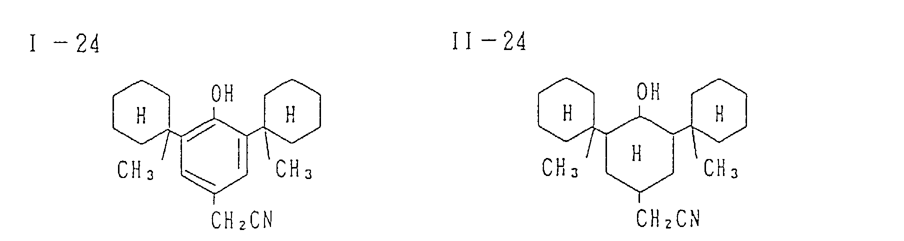

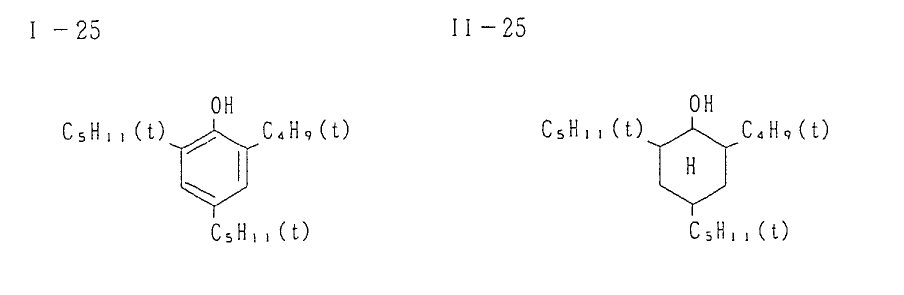

A rhodium or ruthenium catalyst which is used in the present invention may be metal themselves or compounds thereof, and a metal in a fine powder or colloid form carried on a carrier is preferably used. Types of carrier are not specifically restricted, but activated carbon or alumina is preferably used. A suitable amount of rhodium or ruthenium carried on a carrier is generally 0.5 to 20% by weight based on the amount of the catalyst, but is not restricted thereto. The rhodium and ruthenium catalyst used in the present invention are available from N.E.CHEMCAT CORPORATION or Aldrich Chemical Company, Inc.

Preference is given to the use of a large amount of catalyst, because the larger the amount of the catalyst, faster the reaction speed is. The amount of the catalyst is preferably 0.5 to 200% by weight, more preferably 1 to 150% by weight based on the raw material, but it is needless to say that the amount is varied depending upon the content of rhodium or ruthenium in the catalyst.

Examples of solvents which can be used for the catalytic hydrogeneration reaction include alcohols such as methanol, ethanol and isopropanol, esters such as ethyl acetate, ethers such as tetrahydrofuran, acetic acid, and water. A mixture of these solvent may be used, but it is most preferable to carry out the reaction without solvent. Even if the raw material is solid under the normal temperature and normal pressure, the reaction can be carried out without solvent by melting the raw material under the reaction conditions described in some of the existing patents. Depending upon the raw material and the reaction conditions, in some cases, the reaction to a cyclohexanol only proceeds under the condition in the absence of solvent, and if a solvent is added, the reaction is stopped when a cyclohexanol is formed. In addition, in the case of the reaction in the absence of solvent, since the amount to be put in an autoclave as a raw material is large in comparison with the case of using a solvent in the same autoclave having the same volume, it is suitable for producing a product in a large amount. Furthermore, the reaction in the absence of solvent is also advantageous in that no solvent cost is required and a post-treatment can be simplified.

In order to hydrogenate 2,6-di-tert-alkylphenol of formula (I) to obtain 2,6-di-tert-alkylcyclohexanol of formula (II), the raw material and the above-mentioned rhodium or ruthenium catalyst are incorporated in a pressure vessel, and the raw material is reacted with hydrogen under a hydrogen pressure by heating and stirring the system. In this case, the higher the reaction temperature, faster the reaction speed is, which shortens the period of completing the reaction. However, in this case, the alcohol formed is further decomposed by the hydrogenation to decrease the yield. The reaction temperature is, therefore, preferably from 50°C to 300°C, more preferably from 60°C to 250°C, and particularly from 80°C to 240°C. The hydrogen pressure in the vessel is not specifically restricted, but a high hydrogen pressure is preferable. Typically, the hydrogen pressure is preferably from 10 kg/m² to 250 kg/m², and more preferably from 15 kg/m² to 200 kg/m², but is not specifically restricted thereto.

The present invention will now be described in greater detail with reference to the following examples, but the present invention is not restricted thereto.

### EXAMPLE 1 (Catalytic Hydrogenation)

Into 500 mℓ volume stainless-made autoclave were put 100 g of 2,6-di-tert-butyl-4-methylphenol and 5 g of 5%-rhodium/alumina catalyst produced by N.E.CHEMCAT CORPORATION, the autoclave was sealed, the atmosphere was replaced by hydrogen so that an initial hydrogen pressure was set at 100 kg/m², and the system was stirred at 200°C for 12 hours. After cooling down to room temperature, the reaction product was taken, and the catalyst was filtered off. When the resulting product was quantitatively determined by a gas chromatography, it was found to be 2,6-di-tert-butyl-4-methylcylohexanol having a purity of 95%. The construction was identified by NMR spectrum.

| | |
|---|---|
| Boiling point | 95°C/2 mmHg |
| Density | 0.776 g/cm³ (25°C) |
| Viscosity | 15.6 cp (25°C) |
| Refractive index | 1.4630 (25°C) |
| Dielectric constant | 3.89 (25°C) |

NMR: 0.95 ppm, 18H (s); 4.4 ppm 1H (s).

When the hydrogenation was carried out under the same conditions as described above using the same raw material except for changing the catalyst to 5 g of 5% ruthenium/alumina catalyst produced by Aldrich Chemical Company, Inc., 2,6-di-tert-butyl-4-methylcylohexanol was similarly obtained in a high yield.

In addition, when similar reactions were carried out for comparison, changing the catalyst to palladium/activated carbon, platinum/activated carbon, platinum dioxide, Raney-nickel catalysts, etc., in all cases, the product was 2,6-di-tert-butyl-4-methylcyclohexanone, but no 2,6-di-tert-butyl-4-methylcylohexanol was produced.

The results are summarized in Table 1.

**Table 1**

| | Raw material | Type of Catalyst | Amount of catalyst (g) | Reaction period (hr) | Ratio of productions, cylcohexanon e/cyclohexan ol |
|---|---|---|---|---|---|
| 1 | I-1 | 5%-Rh/Alumina powder | 5 | 12 | 2/98 |
| 2 | " | 5%-Rh/Carbon powder | 5 | 12 | 2/98 |
| 3 | " | 5%-Ru/Carbon powder | 5 | 12 | 2/98 |
| 4 | " | 5%-Ru/Alumina powder | 5 | 12 | 2/98 |
| 5 | " | 5%-Rh/Alumina powder | 10 | 6 | 2/98 |
| 6 | " | 5%-Pd/Carbon powder | 10 | 12 | 100/0 |
| 7 | " | PtO₂ powder | 2 | 12 | 100/0 |
| 8 | " | 5%-Pt/Carbon powder | 5 | 12 | 100/0 |
| 9 | " | Raney Ni | 5 | 12 | 100/0 |
| 10 | I-2 | 5%-Rh/Alumina powder | 5 | 12 | 5/95 |
| 11 | I-3 | 5%-Rh/Alumina powder | 5 | 12 | 5/95 |
| 12 | I-4 | 5%-Rh/Alumina powder | 5 | 12 | 5/95 |
| 13 | I-5 | 5%-Rh/Alumina powder | 5 | 12 | 5/95 |

As is clear from Table 1, according to the present invention, 2,6-di-tert-alkylcyclohexanol of formula (II) can be synthesized by catalytically hydrogenating 2,6-di-tert-alkylphenol of formula (I) with high effectiveness using rhodium or ruthenium as a catalyst.

According to the present invention, a 2,6-di-tert-alkylcyclohexanol can be effectively synthesized.

## Claims

1. A process for producing a 2,6-di-tert-alkylcyclohexanol represented by formula (II) comprising catalytically hydrogenating a 2,6-di-tert-alkylphenol represented by formula (I) in the presence of a rhodium or ruthenium catalyst: 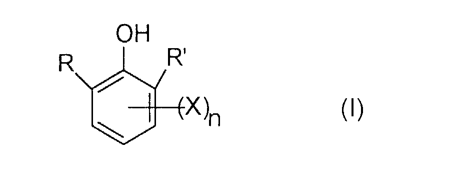 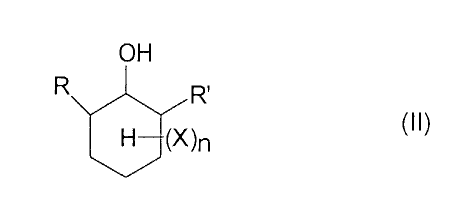 wherein R and R' are independently represented by formula (III) 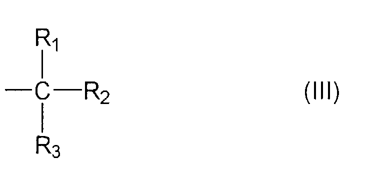 wherein R₁, R₂ and R₃ may be the same or different and represent a substituent, which may be connected to each other to form a ring, X represents a substituent, and n is an integer from 0 to 3.

2. The process as claimed in Claim 1, wherein the catalytic hydrogenation reaction is carried out in the absence of any solvent.

3. The process as claimed in Claim 1 or 2, wherein the catalytic hydrogenation reaction is carried out at a temperature of from 80°C to 240°C.

4. The process as claimed in any of Claims 1 to 3, wherein the catalytic hydrogenation reaction is carried out in a reactor at a hydrogen pressure of from 15 kg/cm² to 200 kg/cm².

5. The process as claimed in any of Claims 1-4, wherein X represents an aliphatic group, an aryl group, a heterocyclic group, an alkoxy group, an aryloxy group, a heterocyclic oxy group, an alkylacyloxy group, an arylacyloxy group, a heterocyclic acyloxy group, a carbamoyloxy group, a silyloxy group, an acylamino group, an alkylamino group, an arylamino group, an ureido group, an alkenyloxy group, a formyl group, an alkylacyl group, an arylacyl group, a heterocyclic acyl group, an alkyloxycarbonyl group, an aryloxycarbonyl group, a heterocyclic oxycarbonyl group, an alkyloxycarbonylamino group, an aryoxycarbonylamino group, a heterocyclic oxycarbonylamino group, a carbamoyl group, a phosphonyl group, an imide group, an azolyl group, a halogen atom, a hydroxy group, a cyano group, a carboxy group, a nitro group or an unsubstituted amino group.

6. The process as claimed in any of Claims 1-5, wherein R₁, R₂ and R₃ are independently an aliphatic group, an aryl group, a heterocyclic group, a formyl group, an alkylacyl group, an arylacyl group, a heterocyclic acyl group, an alkyloxycarbonyl group, an aryloxycarbonyl group, a heterocyclic oxycarbonyl group, a carbamoyl group or a carboxyl group.

7. The process as claimed in Claim 6, wherein R₁, R₂ and R₃ are independently a straight or branched chain alkyl group having 1 to 15 carbon atoms, an aralkyl group having 7 to 15 carbon atoms, a cycloalkyl group having 3 to 15 carbon atoms, or an aryl group having 6 to 15 carbon atoms.

8. The process as claimed in Claim 7, wherein R₁, R₂ and R₃ are independently a straight or branched chain alkyl group having 1 to 8 carbon atoms, an aralkyl group having 7 or 8 carbon atoms, a cycloalkyl group having 5 to 8 carbon atoms, or an aryl group having 6 to 8 carbon atoms,

9. The process as claimed in any of Claims 1-8, wherein n is 1.

10. The process as claimed in Claim 6, wherein R₁, R₂ and R₃ are independently an alkyl group having 1 to 15 carbon atoms, an aralkyl group having 7 to 15 carbon atoms, a cycloalkyl group having 3 to 15 carbon atoms, or an aryl group having 6 to 15 carbon atoms; X is an alkyl group having 1 to 15 carbon atoms, an aralkyl group having 7 to 15 carbon atoms, a cycloalkyl group having 3 to 15 carbon atoms, or an aryl group having 6 to 15 carbon atoms; n is 1; and X exists on the para position to the hydroxyl group of formulae (I) and (II).

11. The process as claimed in Claim 1, wherein both of R and R' are a tert-butyl group, a tert-amyl group, a tert-octyl group or a 1-methylcyclohexyl group; X is a methyl group, an ethyl group, an n-propyl group, an iso-propyl group, an n-butyl group, a sec-butyl group, a tert-amyl group, a tert-octyl group, a phenyl group or a cyclohexyl group; n is 1; and X exists on the para position to the hydroxyl group of formulae (I) and (II).

## Patentansprüche

1. Verfahren zur Herstellung eines 2,6-Di-tert-alkylcyclohexanols der Formel (II), umfassend die katalytische Hydrierung eines 2,6-Di-tert-alkylphenols der Formel (I) in Gegenwart eines Rhodium- oder Ruteniumkatalysators: 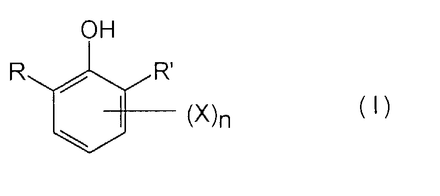 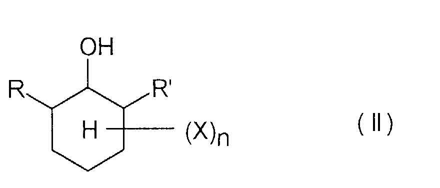 worin R und R' unabhängig voneinander durch die Formel (III) repräsentiert werden: 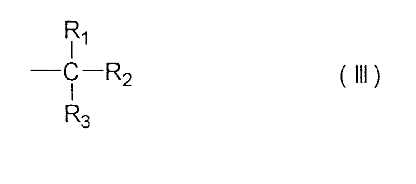 worin R₁, R₂ und R₃ identisch oder voneinander verschieden sind und einen Substituenten repräsentieren, die unter Bildung eines Ringes miteinander verbunden sein können, X repräsentiert einen Substituenten und n ist eine ganze Zahl von 0 bis 3.

2. Verfahren gemäss Anspruch 1, worin die katalytische Hydrierungsreaktion in Abwesenheit jeglichen Lösungsmittels durchgeführt wird.

3. Verfahren gemäss Anspruch 1 oder 2, worin die katalytische Hydrierungsreaktion bei einer Temperatur von 80 bis 240°C durchgeführt wird.

4. Verfahren gemäss einem der Ansprüche 1 bis 3, worin die katalytische Hydrierungsreaktion in einem Reaktor bei einem Wasserstoffdruck von 15 bis 200 kg/cm² durchgeführt wird.

5. Verfahren gemäss mindestens einem der Ansprüche 1 bis 4, worin X eine aliphatische Gruppe, eine Arylgruppe, eine heterocyclische Gruppe, eine Alkoxygruppe, eine Aryloxygruppe, eine heterocyclische Oxygruppe, eine Alkylacyloxygruppe, eine Arylacyloxygruppe, eine heterocyclische Acyloxygruppe, eine Carbamoyloxygruppe, eine Silyloxygruppe, eine Acylaminogruppe, eine Alkylaminogruppe, eine Arylaminogruppe, eine Ureidogruppe, eine Alkenyloxygruppe, eine Formylgruppe, eine Alkylacylgruppe, eine Arylacylgruppe, eine heterocyclische Acylgruppe, eine Alkoxycarbonylgruppe, eine Aryloxycarbonylgruppe, eine heterocyclische Oxycarbonylgruppe, eine Alkyloxycarbonylaminogruppe, eine Aryloxycarbonylaminogruppe, eine heterocyclische Oxycarbonylaminogruppe, eine Carbamoylgruppe, eine Phosphonylgruppe, eine Imidgruppe, eine Azolylgruppe, ein Halogenatom, eine Hydroxygruppe, eine Cyanogruppe, eine Carboxygruppe, eine Nitrogruppe oder eine unsubstituierte Aminogruppe repräsentiert.

6. Verfahren gemäss mindestens einem der Ansprüche 1 bis 5, worin R₁, R₂ und R₃ unabhängig voneinander eine aliphatische Gruppe, eine Arylgruppe, eine heterocyclische Gruppe, eine Formylgruppe, eine Alkylacylgruppe, eine Arylacylgruppe, eine heterocyclische Acylgruppe, eine Alkoxycarbonylgruppe, eine Aryloxycarbonylgruppe, eine heterocyclische Oxycarbonylgruppe, eine Carbamoylgruppe oder eine Carboxylgruppe darstellen.

7. Verfahren gemäss Anspruch 6, worin R₁, R₂ und R₃ unabhängig voneinander eine verzweigte oder unverzweigte Alkylgruppe mit 1 bis 15 Kohlenstoffatomen, eine Aralkylgruppe mit 7 bis 15 Kohlenstoffatomen, eine Cycloalkylgruppe mit 3 bis 15 Kohlenstoffatomen oder eine Arylgruppe mit 6 bis 15 Kohlenstoffatomen sind.

8. Verfahren gemäss Anspruch 7, worin R₁, R₂ und R₃ unabhängig voneinander eine unverzweigte oder verzweigte Alkylgruppe mit 1 bis 8 Kohlenstoffatomen, eine Aralkylgruppe mit 7 oder 8 Kohlenstoffatomen, eine Cycloalkylgruppe mit 5 bis 8 Kohlenstoffatomen oder eine Arylgruppe mit 6 bis 8 Kohlenstoffatomen darstellen.

9. Verfahren gemäss mindestens einem der Ansprüche 1 bis 8, worin n 1 ist.

10. Verfahren gemäss Anspruch 6, worin R₁, R₂ und R₃ unabhängig voneinander eine Alkylgruppe mit 1 bis 15 Kohlenstoffatomen, eine Aralkylgruppe mit 7 bis 15 Kohlenstoffatomen, eine Cycloalkylgruppe mit 3 bis 15 Kohlenstoffatomen oder eine Arylgruppe mit 6 bis 12 Kohlenstoffatomen sind; X ist eine Alkylgruppe mit 1 bis 15 Kohlenstoffatomen, eine Aralkylgruppe mit 7 bis 15 Kohlenstoffatomen, eine Cycloalkylgruppe mit 3 bis 15 Kohlenstoffatomen oder eine Arylgruppe mit 6 bis 15 Kohlenstoffatomen; n ist 1; und X befindet sich in der para-Position zu der Hydroxylgruppe der Formeln (I) und (II).

11. Verfahren gemäss Anspruch 1, worin R und R' beide eine tert-Butylgruppe, eine tert-Amylgruppe, eine tert-Octylgruppe oder eine 1-Methylcyclohexylgruppe sind; X ist eine Methylgruppe, eine Ethylgruppe, eine n-Propylgruppe, eine Isopropylgruppe, eine n-Butylgruppe, eine sek-Butylgruppe, eine tert-Amylgruppe, eine tert-Octylgruppe, eine Phenylgruppe oder eine Cyclohexylgruppe; n ist 1; und X befindet sich in der para-Position zu der Hydroxylgruppe der Formeln (I) und (II).

## Revendications

1. Procédé de production de 2,6-di-tert-alkylcyclohexanol représenté par la formule (II), comprenant l'hydrogénation catalytique d'un 2,6-di-tert-alkylphénol représenté par la formule (I) en présence d'un catalyseur de rhodium ou de ruthénium: 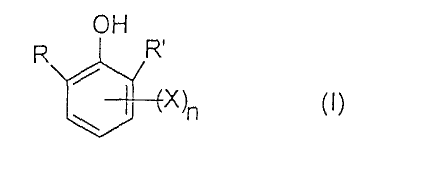 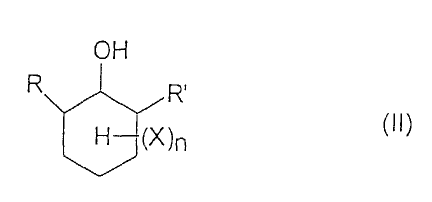 dans lesquelles R et R' sont représentés indépendamment par la formule (III) 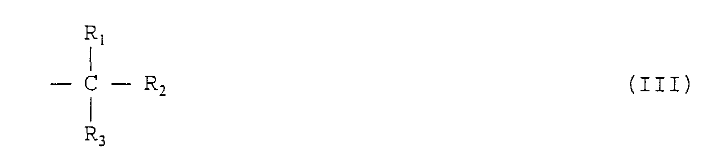 dans laquelle R₁, R₂ et R₃ peuvent être identiques ou différents et représentent un substituant, qui peuvent être relié ensemble pour former un cycle, X représente un substituant, et n est un entier de 0 à 3.

2. Procédé selon la revendication 1, dans lequel la réaction d'hydrogénation catalytique est mise en oeuvre en l'absence de tout solvant.

3. Procédé selon la revendication 1 ou 2, dans lequel la réaction d'hydrogénation catalytique est mise en oeuvre à une température de 80°C à 240°C.

4. Procédé selon l'une quelconque des revendications 1 à 3, dans lequel la réaction d'hydrogénation catalytique est mise en oeuvre dans un réacteur à une pression d'hydrogène de 15 kg/cm² à 200 kg/cm².

5. Procédé selon l'une quelconque des revendications 1-4, dans lequel X représente un groupe aliphatique, un groupe aryle, un groupe hétérocyclique, un groupe alcoxy, un groupe aryloxy, un groupe oxy hétérocyclique, un groupe alkylacyloxy, un groupe arylacyloxy, un groupe acyloxy hétérocyclique, un groupe carbamoyloxy, un groupe silyloxy, un groupe acylamino, un groupe alkylamino, un groupe arylamino, un groupe uréido, un groupe alcényloxy, un groupe formyle, un groupe alkylacyle, un groupe arylacyle, un groupe acyle hétérocyclique, un groupe alkyloxycarbonyle, un groupe aryloxycarbonyle, un groupe oxycarbonyle hétérocyclique, un groupe alkyloxycarbonylamino, un groupe aryloxycarbonylamino, un groupe oxycarbonylamino hétérocyclique, un groupe carbamoyle, un groupe phosphonyle, un groupe imide, un groupe azolyle, un atome d'halogène, un groupe hydroxy, un groupe cyano, un groupe carboxy, un groupe nitro ou un groupe amino non-substitué.

6. Procédé selon l'une quelconque des revendications 1-5, dans lequel R₁, R₂ et R₃ représentent indépendamment un groupe aliphatique, un groupe aryle, un groupe hétérocyclique, un groupe formyle, un groupe alkylacyle, un groupe arylacyle, un groupe acyle hétérocyclique, un groupe alkyloxycarbonyle, un groupe aryloxycarbonyle, un groupe oxycarbonyle hétérocyclique, un groupe carbamoyle ou un groupe carboxyle.

7. Procédé selon la revendication 6, dans lequel R₁, R₂ et R₃ représentent indépendamment un groupe alkyle à chaîne droite ou ramifiée présentant de 1 à 15 atomes de carbone, un groupe aralkyle présentant de 7 à 15 atomes de carbone, un groupe cycloalkyle présentant de 3 à 15 atomes de carbone, ou un groupe aryle présentant de 6 à 15 atomes de carbone.

8. Procédé selon la revendication 7, dans lequel R₁, R₂ et R₃ représentent indépendamment un groupe alkyle à chaîne droite ou ramifiée présentant de 1 à 8 atomes de carbone, un groupe aralkyle présentant 7 ou 8 atomes de carbone, un groupe cycloalkyle présentant 5 à 8 atomes de carbone ou un groupe aryle présentant 6 à 8 atomes de carbone.

9. Procédé selon l'une quelconque des revendications 1-8, dans lequel n vaut 1.

10. Procédé selon la revendication 6, dans lequel R₁, R₂ et R₃ représentent indépendamment un groupe alkyle présentant 1 à 15 atomes de carbone, un groupe aralkyle présentant 7 à 15 atomes de carbone, un groupe cycloalkyle présentant 3 à 15 atomes de carbone ou un groupe aryle présentant 6 à 15 atomes de carbone; X est un groupe alkyle présentant 1 à 15 atomes de carbone, un groupe aralkyle présentant 7 à 15 atomes de carbone, un groupe cycloalkyle présentant 3 à 15 atomes de carbone ou un groupe aryle présentant 6 à 15 atomes de carbone; n vaut 1; et X existe dans la position para sur le groupe hydroxyle des formules (I) et (II).

11. Procédé selon la revendication 1, dans lequel R et R' représentent tous deux un groupe tert-butyle, un groupe tert-amyle, un groupe tert-octyle ou un groupe 1-méthylcyclohexyle; X représente un groupe méthyle, un groupe éthyle, un groupe n-propyle, un groupe iso-propyle, un groupe n-butyle, un groupe sec-butyle, un groupe tert-amyle, un groupe tert-octyle, un groupe phényle ou un groupe cyclohexyle; n vaut 1; et X existe dans la position para sur le groupe hydroxyle des formules (I) et (II).
